# EUROPEAN PATENT APPLICATION

(11) **EP 1 647 245 A2**
(43) Date of publication of application: **19.04.2006**
(21) Application number: 05425691.2
(22) Date of filing: 05.10.2005
(51) Int. Cl.: A61F 5/00, A61F 2/50

(54) **Adhesive self-supporting epithesis/prothesis with plates of interface**

(30) Priority: 14.10.2004 IT VT20040006
(71) Applicant: PAOLOCCI, Alfredo, 01030 Vasanello VT (IT)
(72) Inventor: PAOLOCCI, Alfredo, 01030 Vasanello VT (IT)

(57) **Abstract**

Application in the field of medicine and more precisely in the sector of prosthetic rehabilitation concerning a new type of external epitheses/prostheses of the face and body fixed to the patient through an adhesive put on the plates of interface.

## Description

Industrial invention entitled: **"adhesive self-supporting** epithesis/prosthesis with plates of interface" created to be used in the prosthetic rehabilitation after agenesis or damage to the facial features or to another part of the body, destroyed, damaged and/or excised because of tumoral pathologies, burns or after trauma/accidents.

That is namely **"Innovation in the field of medicine and more** precisely in the sector of prosthetic rehabilitation concerning a new type of external epitheses/prostheses of the face and body **fixed to the patient through an adhesive put on the plates of interface"** of Mr.: Paolocci Alfredo - born in Orte on August 19^{th}, 1935 - resident in Vasanello Postcode 01030 (VT) Via Palazzolo n.142 - Locality of scopiglieto .

The objective of the above-mentioned invention concerns a new **type of maxillofacial epitheses or prostheses** conceived in an innovative way to be better fixed to the body of the patient, however it also develops and includes any other type of prostheses.

We believe it is proper to talk about "a new type of epitheses/prostheses" since from their conceiving they are different from those used nowadays both in their structure and in better adhesive efficiency and fixing to the patient.

So far the techniques used for the anchorage of the large size epitheses to the cranium and to the face have always expected a surgical operation on the patient consisting in the insertion of precise retentive implants in the available part of the bone (the so called extraoral and/or magnetic implanting system). This fact always implies an accurate care and cleaning of the cutis in the involved part of the body, where the metal protrusions of the several anchorages are placed; in fact, the cleaning has not to be undervalued because the risks of infection and of rejection of such implants are very high.

Planned and created with our peculiar method, this new type of epitheses which excludes any type of risks - and it becomes the crucial solution - is used especially in those cases where the insertion of craniofacial implants used as anchorages to fix the normal maxillofacial epitheses is actually impossible, or when the patient explicitly requires for this invention rather than the implanting prostheses. This device has the purpose to help fastly, as soon as possible, all those patients who need to find again their psychological tranquillity, lost after the unlucky event, that caused the damage of their features (that is to say their changed appearance), and unfortunately they often end up getting used to withdraw into a life without social and familiar relationships.

Moreover, these innovative epitheses are very useful when, after particularly wide and deep bums, it is not possible to undergo the patient to reconstructive surgical operations of the damaged or lost part. The first and fundamental feature of this device is to let a strong and safe fixing to the body, without precedent, through **the only adhesive,** without the use of anchorages and surgical implants on the patient or external systems of any kind to these epitheses/prostheses and/or other visible and foreign body to the same prosthetic handmade product. We go on the description with the help of drawings and pictures: the invention, which from now on we are going to call < **adhesive self-supporting** epithesis/prosthesis with plates of interface > is essentially made of two main elements or parts: a first part is represented by a *handmade product of silicone* which reproduces the organ or the rebuilt tissue, a second part made of a *surface*/*plate of different material* suitable for receiving the adhesive and from now on we are going to call "plate of interface". Our invention develops and improves also the way to use the adhesive for the epitheses, which is no more put on the silicone, thanks to our device, rather than on an alternative material, suitable for the purpose.

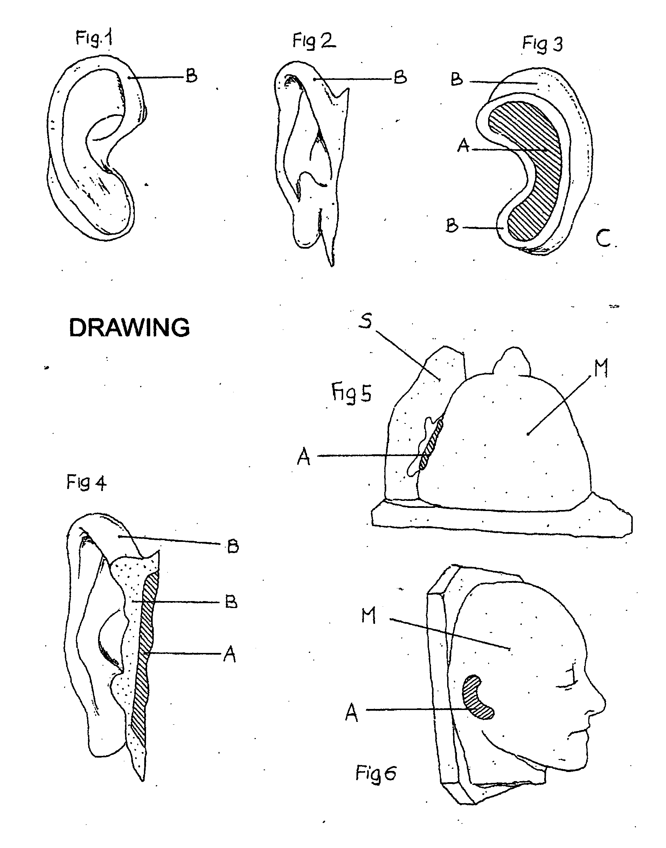

First of all the epithesis or prosthesis adheres exactly and complementary to the body of the patient because it is shaped in laboratory with his exact information, referring to the study of his face and/or his body, it is produced with suitable materials and sticked through a **compatible adhesive** to the involved part (such as: biological glues, medical adhesives, adhesives for maxillofacial epitheses etc). In our invention the adhesive is put on the surface of the plate of interface (A) which will be able to have different shape and size and it will be placed in variable quantities according to the cases. The above-mentioned **plate (Fig. 3,4,5,6)** will be always placed on the side of the prosthesis that comes into contact with the patient (side C , Fig 3).

Thanks to the use of the rigid or semi-rigid **plates of interface** we have seen the exponential and considerable growth of the adhesive and retentive power of the glue normally used for the epitheses.

Before our invention, the glue which was put directly on the silicone of the maxillofacial epitheses was useful for few gram small epitheses but ineffective and inadequate for significant and wide epitheses.

**The efficiency of these new epitheses is remarkable** and let that these handmade products are sticked to the face or to the body without moving, after their placing, even for many hours.

This new type of epithesis is also daily removable as it happened for the normal maxillofacial epitheses.

The part is shaping according to the photographic documents before the event that caused the destruction of the patient's features and thanks to the qualified competence and sculptural-artistic skill of those in charge of modelling (anaplastologist).

Our invention is also suitable for the standard prostheses (that is to say not customized), conceived and made, without knowing the patient, on a large scale, on an industrial level, without changing its efficiency or validity, such as the fingers of the hand, the ear, the breast of a woman or the partial or total prosthesis of a limb.

### Procedure to create an "adhesive self-supporting epithesis with plates of interface".

### We are going to explain the invention choosing the making of an auricular epithesis.

We go on the description with the help of the drawings and the enclosed pictures:
**Fig. 1** shows the adhesive epithesis in the frontal version, in which only the silicone part **B** that reproduces the rebuilt organ is visible,
**Fig.** 2 shows the profile of the adhesive epithesis, in which only the silicone part **B** that reproduces the rebuilt organ is visible,
**Fig.** 3 shows the backside of the adhesive epithesis (side C of the epithesis) where the plate **A** of interface is clearly visible,
**Fig. 4** shows especially the several layers of the materials, through a transversal section, and the different components that define the epithesis: the silicone part **B** and the plate **A** of interface,
**Fig.** 5 shows especially the exact position of the moulds **M** and **S,** that close for the creation of the epithesis,
**Fig.** 6 shows especially the plate A of interface made on the model **M** of the patient,

We also enclose **explanatory pictures** of the prosthesis made with the same numbers and letters of the drawing. Among the several materials used for the creation of the prosthesis, objective of the current patent, we point out those tested and used for the creation of maxillofacial epitheses such as the addition-vulcanizing silicone and similar products of physiological high quality, and the relative medical adhesives.

### Creation of the patient's model, photographic and colour surveys.

During this preliminary and more difficult phase, it is necessary an accurate study on the subject: all the colour surveys of the epidermis, comparisons and evaluation of the volume and the surface to rebuilt totally or partially and a detailed photographic and videotaped documentation.

**We create - a mould - on the patient** of the area that has undergone the excision.

### Creation in the laboratory of the model of the patient:

We develop the **positive (M) into plaster** of the imprinted part (master model) pouring the extra strong plaster into the negative mould, that is to say into the print just obtained from the patient, making the so called "positive" **(M)** of the patient **(Fig. 5,6)**

**We make the plate of interface A** with the suitable materials on the model M of the patient **(Fig.** 6).

**We create the new features** with the modelling wax; the epithesis will take shape carrying out a reconstruction, right on the plate made previously, placing on model M of the patient.

**We create the mould of the modelled part:** we make a mould or a negative part **S (Fig.** 5), able to "fix" the exact details of the modelled part and which contains all the aesthetic-morphologic features of the patient's missing part; this fact lets the "reproduction" of the last one (in case of copies) repeatedly and at different times, in its every small survey, without creating differences between the first extruded product and the following ones.

The next phases consist in :

Distributing the silicone of the same base shade of the patient's skin, inside the mould S, properly cleaned up from the traces of the modelling wax.

**Placing the plate on the model of the patient M** putting it at the exact point where it was shaped **(Fig. 5,6)** after having arranged the proper retention for the anchorage to the silicone.

Closing in a way to press the two moulds **M** and **S,** then we have to wait the reaction of the fixing (placed as in **Fig.** 5).

Opening the moulds **M** and **S:** Extrusion of the handmade product **(Fig. 1,2,3,4)** made of the silicone handmade B, which reproduces the rebuilt part, anchored on a **plate A of interface,** visible from the side **C** of the prosthesis **(Fig.** 3) that comes into contact with the patient.

The above-mentioned plate will be responsible for the perfect complementarity of the patient with the epithesis, and just on the surface of the plate that the adhesive will be always put during the use of the epithesis.

**The plates of interface are fundamental for the fixing of the epithesis/prosthesis to the patient.** As we have said previously, they interact with the glue, strengthening the adhesive and fixative power and making more effective the adhesive or the medical glue. Without the plates, materials such as the silicone (for the prostheses/epitheses) and the natural skin of the patient could hardly keep sticked. These plates can be of different shape, composition and size, on the condition that they have to be made in a suitable material, able to determinate the best performance of the adhesive, which can be removable from the cutis and from the prosthesis/epithesis at every moment with a specific solvent.

We achieved the best results creating also the above-mentioned plates in rigid, strong and light materials, such as compounds made of metylmethacrylate.

The number, the size, the colour and the shape of the plates are established by several affecting factors; we mention some examples: the presence of particularly sensitive areas of the patient, the shape and the size of the prosthesis/epithesis to make and then to fix through the above-mentioned plates.

## Claims

1. "adhesive self-supporting epithesis/prosthesis with plates of interface", **characterized by** not using the anchorages or surgical and/or magnetic implants of any kind on the patient for its fixing.

2. "adhesive self-supporting epithesis/prosthesis with plates of interface", according to the previous claim, is **characterized by** the only use of the adhesive system for the fixing to the patient.

3. "adhesive self-supporting epithesis/prosthesis with plates of interface", according to the previous claims, is **characterized by** one or more plates of interface of different composition and size for the fixing to the patient through the only adhesive system.
